# EUROPEAN PATENT SPECIFICATION

(11) **EP 3 463 213 B1**
(45) Date of publication and mention of the grant of the patent: **04.03.2026**
(21) Application number: 17727192.1
(22) Date of filing: 29.05.2017
(51) Int. Cl.: A61F 5/01, A41D 19/015, A61H 1/02, B25J 9/00

(54) **FINGER GLOVE LINER AND STRENGTHENING FINGER GLOVE WITH LINER**
FINGERHANDSCHUHFUTTER UND VERSTÄRKENDER FINGERHANDSCHUH MIT INNENFUTTER
DOUBLURE DE GANT DE DOIGT ET RENFORCEMENT DU GANT DE DOIGT PAR L'INTERMÉDIAIRE DE LA DOUBLURE

(30) Priority: 30.05.2016 SE 1650751
(43) Date of publication of application: 10.04.2019
(73) Proprietor: Bioservo AB, 164 40 Kista (SE)
(72) Inventor: EWALDSSON, Martin, Oskar, Gustaf, 193 40 Sigtuna (SE); FRODE, Lars, 112 25 Stockholm (SE); GABRIELSSON, Carl, Joakim, 183 57 Täby (SE); LINDER, Emma, Helena, 74131 Knivsta (SE)
(74) Representative: Noréns Patentbyrå AB
(86) International application number: PCT/EP2017/062932
(87) International publication number: WO 2017/207507

(56) References cited:
- WO-A1-2008/027002
- US-A1- 2012 029 399
- US-A1- 2013 219 586

## Description

### Technical field

The present invention concerns grip strengthening finger glove having a liner for arrangement inside an outer finger glove, making up a grip strengthening finger glove having at least one finger portion for enhancing the grip for at least one corresponding finger of a wearer by means of at least one artificial tendon arranged along at least one finger portion of the grip strengthening support device.

### Background

There are previously known grip strengthening support devices, for example shown in WO 2008/027002. In this document a finger glove for grip strengthening is shown, having at least one glove finger with at least one artificial tendon on the inside of the glove finger on both sides of the glove finger and the artificial tendon runs in a duct attached on the inside of the glove finger. When a pulling force is applied to the artificial tendon the glove finger will bend towards the palm of the glove, i.e. the ventral side of the glove.

It is cumbersome attaching and arranging the artificial tendons and guiding means therefor on the inside of a finger glove. Additionally, sensors and wires need also to be attached. Another way of arranging and attaching artificial tendons, sensors and wires is to arrange and attach them at a liner which thereafter is covered by a finger glove. It is still a rather work extensive handling much made by hand and in many different sizes, thus causing an expensive product.

Also US 2013/0219586 and US 2012029399 disclose grip strengthening gloves and form part of the prior art, US 2012029399 shows a dual structure in which an outer movement assisting glove and an inner movement assisting glove are sewn together to form a united body and linear members are arranged along the finger parts designed to assist finger movement.

### Summary of the invention

It is an aim of the present invention to provide a grip strengthening finger glove which may be produced in an easier way and partially in less different sizes.

The objective is solved by the invention, which is defined by the claims.

The grip strengthening finger glove defined in claim 1 makes it possible to produce less sizes of the inventive finger glove liner and still provide a suitable fit and the same kind of effective gripping when the at least one artificial tendon is pulled. It is not possible to attach the at least one artificial tendon directly to the stretchable liner material as this would stretch instead of taking up the forces provided by the at least one artificial tendon.

According to one embodiment the at least one patch of material is made of a flexible and non-stretchable material in a direction crosswise the length direction of the finger portion. Thus the at least one patch can distribute the force from the at least one artificial tendon attached to said patch. The force from the artificial tendons will thus be transferred via the patches to a finger of a wearer thus creating a bending force on the finger, which will enhance the gripping of the wearer.

According to one embodiment the patches are arranged freely from each other in order to be able to transfer with the expansion of the stretchable liner in use. In this way the patches will be in the right position even if a bigger hand is put into the stretchable finger glove liner.

According to one embodiment at least one sensor is arranged at at least one patch. Thus the grip strengthening finger glove may sense when a wearer are going to grip something and thus starting the pulling of the at least one artificial tendon in order to enhance the gripping.

According to one embodiment at least one electrical wire is arranged at at least one patch. The electrical wiring may, for example, connect a sensor and an actuator for pulling of the at least one artificial tendon.

According to a second aspect of the invention a grip strengthening finger glove having a finger glove liner according to any of the above embodiments is provided.

### Short description of the drawings

The present invention will now be described in more detailed under referral to the attached drawings, in which:
Fig. 1 shows an embodiment of a finger portion of a finger glove liner.
Fig. 2 shows an embodiment of a dorsal side of a finger glove liner.
Fig. 3 shows an embodiment of a ventral side of a finger glove liner.
Fig. 4 shows an embodiment of a grip strengthening finger glove.

### Detailed description of embodiments

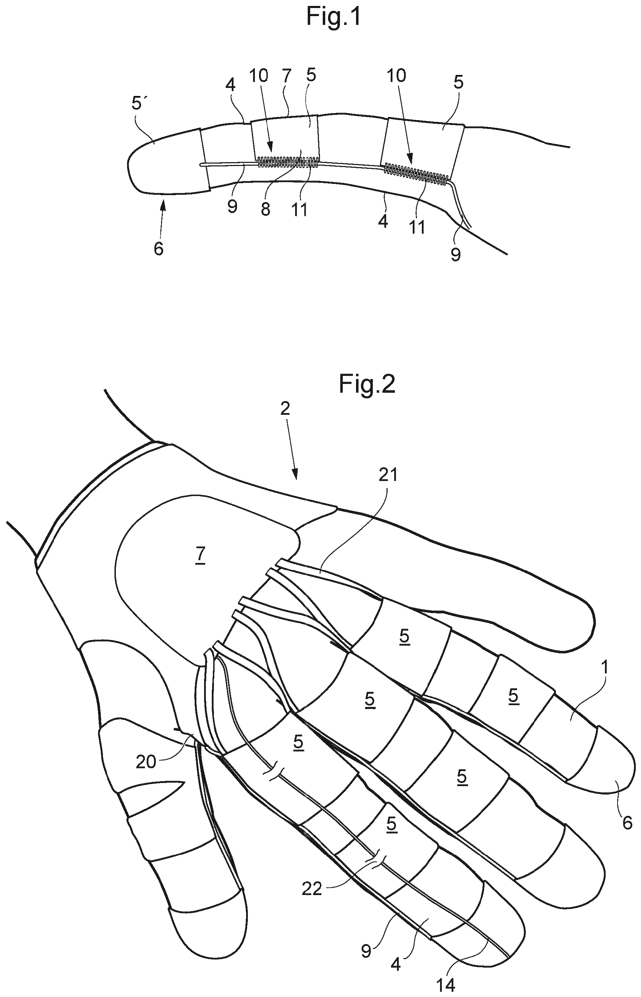

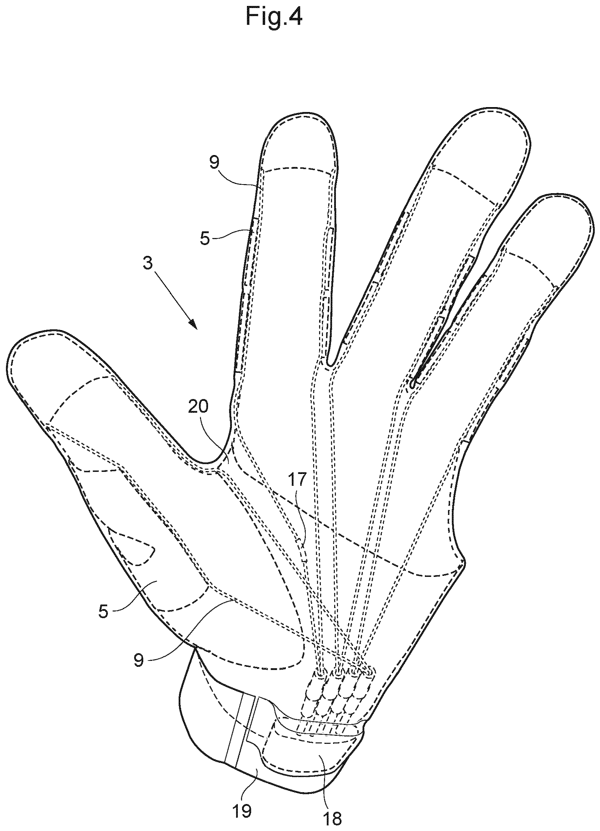

In Fig. 1 a finger portion 1 of a finger glove liner 2 is shown. The finger portion 1 may be suitable for any of the fingers of a wearer's hand including the thumb. A finger glove liner 2 comprises at least one finger portion 1. The finger glove liner 2 is supposed to be provided inside a finger glove 3 making up a grip strengthening finger glove, see Fig. 4.

The finger portion 1 and the finger glove liner 2 comprises a support material 4, which is stretchable. Thus one size of a finger glove liner 2 suits two or preferably three sizes of hands. For example, the material 4 may be a stitched material in order to provide the stretchability. Outside of the stretchable support material 4 at least one patch 5 is arranged. The patch 5 is preferably made of a material which is flexible and non-stretchable in a direction crosswise the length direction of the finger portion 1. In Fig. 1 a tip portion patch 5' over a tip portion 6 and two dorsal patches 5 along the length of the finger portion 1 are arranged. Preferably the dorsal patches 5 are arranged on a dorsal side 7 of the finger portion 1. It is preferred that the dorsal patches 5 reach down on both sides of the finger portion 1. Patches 5 may be provided at further parts of the finger glove liner 2, too, which will be described further below.

At least one artificial tendon 9 is attached at at least one patch 5. In the shown embodiment of Fig. 1 the artificial tendon 9 is slidably attached at the tip portion patch 5' at the tip portion 6 and along the side 8 portion of the dorsal patches 5 along the length of the finger portion 1. The artificial tendon 9 may be attached by means of, for example, loops (not shown) or a tunnel 10, such as a tunnel made up by stitches 11, which is shown in Fig. 1.

The patches 5 are arranged at the stretchable material 4 in such a way that when the stretchable material 4 stretches, the patches 5 will move to a proper position also for a larger hand than a hand fitting within the finger glove liner 2. This is possible when the patches 5 are only attached at the stretchable material 4 at points. In the non-stretched situation, the non-stretchable patches 5 are attached in bulky way such that the length of the patches 5 curves above the stretchable material 4. Thus, when the stretchable material 4 is stretched the patches 5 will straighten out and be flat along the surface of the stretchable material 4. When stretched even further, there will be spaces between patches.

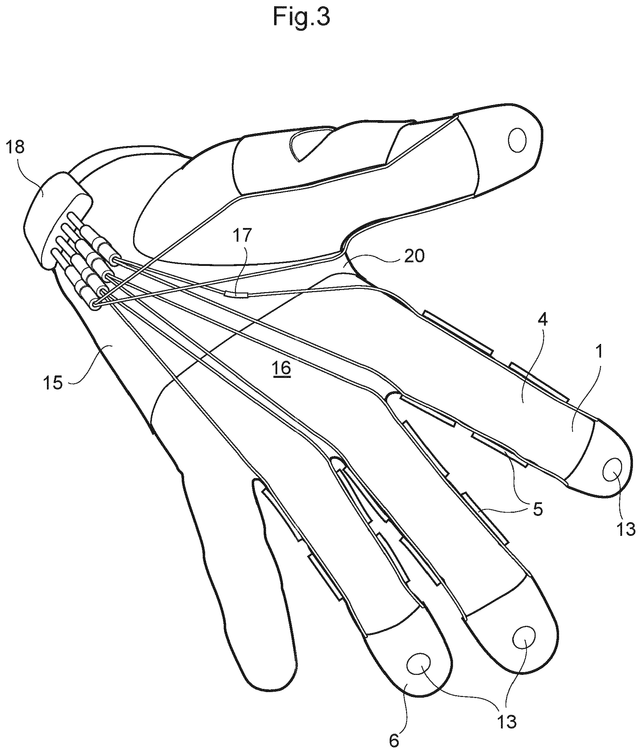

In Figs. 2 and 3 an embodiment of a finger glove liner 2 is shown. Preferably at least one patch 5 is arranged on a ventral side 15 of the finger glove liner 2 at a hand portion 16. This ventral side 15 patch 5 has a guiding 17 for the at least one artificial tendon 9. The ventral side 15 patch 5 may be connected with a patch 5 arranged on the dorsal side 7 of the hand portion 16 of the finger glove liner 2. The connection 20 between the ventral side 15 patch 5 and the dorsal side 7 patch may be stretchable. Preferably a proximal patch 5 at the finger portion 1 is connected with means 21 at the dorsal side 7 patch 5 in order to inhibit sliding of the proximal patch 5 too long towards the distal end of the finger portion 1.

The at least one artificial tendon 9, in the shown embodiment two artificial tendons 9 run along each finger portion 1 possibly into a guiding 17, which guides the artificial tendons 9 towards an actuator 18 for example arranged at a wrist portion 19 of the grip strengthening finger glove 3, see Fig. 4.

The actuator 18 can pull the artificial tendons 9 when being given a signal. The signal will be achieved from at least one sensor 13 arranged in the finger glove liner 2. It is preferred that the at least one sensor 13 is attached at the tip portion 6, preferably at the patch 5 there. Obviously, sensors 13 may be arranged at more positions of the finger glove liner. At least one electrical wire 14 is attached, for example for connection of the at least one sensor 13 with a control means (not shown). In Fig. 2 a possible way of attaching the wire 14 is shown. Slits 22 are provided in the patches 5 and the wire 14 is threaded through the slits 22.

In the present application the embodiments in Figs. 2-4 show a grip strengthening finger glove 3 for all fingers except the little finger, including the thumb. Although it is possible to provide a grip strengthening finger glove 3 having all fingers and strengthening arrangement for all fingers, or only one finger, or the thumb and the index finger, or the thumb and the two middle fingers, or in any other combination.

## Claims

1. A grip strengthening finger glove having an outer finger glove (3) and a finger glove liner (2) comprising at least one finger portion (1), for arrangement inside the outer finger glove (3), wherein an at least one dorsal patch (5) of material, which has attached thereto at least one artificial tendon (9), is arranged at the outside of the finger glove liner (2), the finger glove liner (2) being made of a stretchable material (4) in order to suit more than one size of hands, wherein a tip portion patch (5') is arranged at a tip portion of said finger portion (1) and the at least one dorsal patch (5) is arranged on a dorsal side (7) of said finger portion (1) of the finger glove liner (2), where the at least one dorsal patch (5) on the dorsal side reaches over each of the sides of said finger portion (1) of the finger glove liner (2) with a side portion (8) on each side of said finger portion (1) and the artificial tendon (9) is slidably attached arranged on each of said side portion (8) of said dorsal patch (5) on the dorsal side and along the length of each side of said finger portion (1) on the outside of the finger glove liner (2).

2. The finger glove liner according to claim 1, wherein the at least one dorsal patch (5) of material is made of a flexible and non-stretchable material in a direction crosswise the length direction of the finger portion 1.

3. The finger glove liner according to claim 1 or 2, wherein the dorsal patch(es) (5) and the tip portion patch (5') are arranged freely from each other in order to be able to transfer with the expansion of the stretchable liner (2) in use.

4. The finger glove liner according to claim 1, 2 or 3, wherein the at least one dorsal patch (5) distributes the force from the at least one artificial tendon (9) attached to said dorsal patch (5).

5. The finger glove liner according to claim 1, wherein the at least one artificial tendon (9) is arranged at a side portion (8), along the edge of the side portion (8) of the dorsal patch (5).

6. The finger glove liner according to anyone of the preceding claims, wherein at least one sensor (13) is arranged at the at least one tip portion patch (5').

7. The finger glove liner according to anyone of the preceding claims, wherein at least one electrical wire (14) is arranged at the at least one tip portion patch (5').

## Patentansprüche

1. Griffverstärkender Fingerhandschuh mit einem äußeren Fingerhandschuh (3) und einem Fingerhandschuhfutter (2), das mindestens einen Fingerabschnitt (1) umfasst, der innerhalb des äußeren Fingerhandschuhs (3) angeordnet ist, wobei mindestens ein dorsaler Aufsatz (5) aus Material, an dem mindestens eine künstliche Sehne (9) befestigt ist, an der Außenseite des Fingerhandschuhfutters (2) angeordnet ist, wobei der Fingerhandschuhfutter (2) aus einem dehnbaren Material (4) hergestellt ist, um für mehr als eine Handgröße geeignet zu sein, wobei ein Aufsatz des Spitzenbereichs (5') an einem Spitzenbereich des Fingerabschnitts (1) angeordnet ist und der mindestens eine dorsale Aufsatz (5) auf einer dorsalen Seite (7) des Fingerabschnitts (1) des Fingerhandschuhfutters (2) angeordnet ist, wobei der mindestens eine dorsale Aufsatz (5) auf der dorsalen Seite über jede der Seiten des Fingerabschnitts (1) des Fingerhandschuhfutters (2) mit einem Seitenabschnitt (8) auf jeder Seite des Fingerabschnitts (1) reicht und die künstliche Sehne (9) verschiebbar an jedem der Seitenabschnitte (8) des dorsalen Aufsatzes (5) auf der Rückseite und entlang der Länge jedes Abschnitts des Fingerabschnitts (1) an der Außenseite des Fingerhandschuhfutters (2) verschiebbar angebracht ist.

2. Fingerhandschuhfutter gemäß Anspruch 1, wobei der mindestens eine dorsale Aufsatz (5) aus einem flexiblen und nicht dehnbaren Material in einer Richtung quer zur Längsrichtung des Fingerabschnitts (1) hergestellt ist.

3. Fingerhandschuhfutter gemäß Anspruch 1 oder 2, wobei der/die dorsale(n) Aufsatz/ Aufsätze (5) und der Aufsatz des Spitzenbereichs (5') frei voneinander angeordnet sind, um sich bei Gebrauch mit der Ausdehnung des dehnbaren Futters (2) mitbewegen zu können.

4. Fingerhandschuhfutter gemäß Anspruch 1, 2 oder 3, wobei der mindestens eine dorsale Aufsatz (5) die Kraft von der mindestens einen künstlichen Sehne (9), die an dem dorsalen Aufsatz (5) befestigt ist, verteilt.

5. Fingerhandschuhfutter gemäß Anspruch 1, wobei die mindestens eine künstliche Sehne (9) an einem Seitenabschnitt (8) entlang der Kante des Seitenabschnitts (8) des dorsalen Aufsatzes (5) angeordnet ist.

6. Fingerhandschuhfutter gemäß einem der vorstehenden Ansprüche, wobei mindestens ein Sensor (13) an dem mindestens einen Aufsatz des Spitzenbereichs (5') angeordnet ist.

7. Fingerhandschuhfutter gemäß einem der vorstehenden Ansprüche, wobei mindestens ein elektrischer Draht (14) an dem mindestens einen Aufsatz des Spitzenbereichs (5') angeordnet ist.

## Revendications

1. Gant de doigt de renforcement de préhension ayant un gant de doigt externe (3) et une doublure de gant de doigt (2) comprenant au moins une partie de doigt (1), pour l'agencement à l'intérieur du gant de doigt externe (3), dans lequel une au moins une pièce dorsale (5) de matériau, qui a, fixé à cette dernière, au moins un tendon artificiel (9), est agencée à l'extérieur de la doublure de gant de doigt (2), la doublure de gant de doigt (2) étant réalisée avec un matériau extensible (4) afin de s'adapter à plus d'une taille de mains, dans lequel une pièce de partie de bout (5') est agencée au niveau d'une partie de bout de ladite partie de doigt (1) et la au moins une pièce dorsale (5) est agencée sur un côté dorsal (7) de ladite partie de doigt (1) de la doublure de gant de doigt (2), où la au moins une pièce dorsale (5) sur le côté dorsal atteint chacun des côtés de ladite partie de doigt (1) de la doublure de gant de doigt (2) avec une partie latérale (8) sur chaque côté de ladite partie de doigt (1) et le tendon artificiel (9) est agencé en étant fixé, de manière coulissante, sur chacune de ladite partie latérale (8) de ladite pièce dorsale (5) sur le côté dorsal et le long de la longueur de chaque côté de ladite partie de doigt (1) à l'extérieur de la doublure de gant de doigt (2).

2. Doublure de gant de doigt selon la revendication 1, dans laquelle la au moins une pièce dorsale (5) de matériau est réalisée avec un matériau flexible et non extensible dans une direction transversale à la direction de longueur de la partie de doigt (1).

3. Doublure de gant de doigt selon la revendication 1 ou 2, dans laquelle la (les) pièce(s) dorsale(s) (5) et la pièce de partie de bout (5') sont agencées librement l'une par rapport à l'autre (les unes par rapport aux autres) afin de permettre le transfert avec l'expansion de la doublure extensible (2) à l'usage.

4. Doublure de gant de doigt selon la revendication 1, 2 ou 3, dans laquelle la au moins une pièce dorsale (5) répartit la force du au moins un tendon artificiel (9) fixé à ladite pièce dorsale (5).

5. Doublure de gant de doigt selon la revendication 1, dans laquelle le au moins un tendon artificiel (9) est agencé au niveau d'une partie latérale (8) le long du bord de la partie latérale (8) de la pièce dorsale (5).

6. Doublure de gant de doigt selon l'une quelconque des revendications précédentes, dans laquelle au moins un capteur (13) est agencé au niveau de la au moins une pièce de partie de bout (5').

7. Doublure de gant de doigt selon l'une quelconque des revendications précédentes, dans laquelle au moins un fil électrique (14) est agencé au niveau de la au moins une pièce de partie de bout (5').
